# EUROPEAN PATENT SPECIFICATION

(11) **EP 1 339 356 B1**
(45) Date of publication and mention of the grant of the patent: **13.04.2005**
(21) Application number: 01992527.0
(22) Date of filing: 30.10.2001
(51) Int. Cl.: A61F 2/06, A61F 2/36, A61L 27/00

(54) **TISSUE-ENGINEERED VASCULAR STRUCTURES**
AUS GEWEBE HERGESTELLTE GEFÄSSSTRUKTUREN
STRUCTURES VASCULAIRES DE SYNTHESE

(30) Priority: 30.10.2000 US 244277 P
(43) Date of publication of application: 03.09.2003
(73) Proprietor: CHILDREN'S MEDICAL CENTER CORPORATION, Boston Massachusetts 02115 (US)
(72) Inventor: BISCHOFF, Joyce, Weston, MA 02493 (US); KAUSHAL, Sunjay, Baltimore, MD 21212 (US); MAYER, John, Wellesley, MA 02481 (US); PERRY, Tjorvi, Ellert, Jamaica Plain, MA 02130 (US)
(74) Representative: Brown, David Leslie
(86) International application number: PCT/US2001/048946
(87) International publication number: WO 2002/035992

(56) References cited:
- WO-A-98/15237
- WO-A-99/45775
- US-A- 5 290 271
- US-A- 5 880 090

## Description

### FIELD OF THE INVENTION

The present invention relates generally to tissue engineering and particularly to tissue engineering of structures that require an endothelial surface such as small diameter blood vessels, trileaflet heart valve conduits and for other vascular structures.

### BACKGROUND OF THE INVENTION

Cardiovascular disease, including coronary artery and peripheral vascular disease, is the leading cause of mortality in the United States.¹ Surgical replacement or bypass surgery is the most common intervention for coronary and peripheral atherosclerotic diseases with 550,000 bypass cases performed per year.¹ Autologous vessels are the preferred replacement grafts for diseased segments smaller than 5 mm in diameter, however that involves invasive arterial or venous biopsies and many patients do not have suitable vessels due to amputation or previous vessel harvest.² Moreover, the thin walls of autologous vessels are frequently damaged during transplantation. An alternative approach for obtaining small diameter vessels and other vascular structures is to tissue-engineer vessels by using scaffolds that are based either on a biodegradable or on a decellularized matrix.

In the past, much effort has focused on endothelializing ePTFE grafts to increase their long-term patency in humans³⁹. Previously, the endothelial source for seeding grafts has relied on obtaining a biopsy from an existing blood vessel, thereby sacrificing healthy vessels for harvesting autologous endothelial cells. It has also been shown that in vitro culture of smooth muscle and endothelial cells seeded on a biodegradable or biological scaffold material produced a cellularized vessel with strong mechanical properties, but these grafts thrombosed shortly after implantation *in vivo*.³⁻⁵ Another approach employed for small diameter vessels is to allow repopulation of the tissue-engineered graft with host cells *in vivo*. A decellularized matrix, based either on intestinal collagen or native vessels, remodeled in vivo with infiltration of host smooth muscle cells and endothelial cells into the matrix and demonstrated good patency *in vivo*.⁶⁻⁸ One limitation of these approaches is that the re-populated endothelial cells were not functional in endothelial-dependent relaxation assays performed on the explanted grafts, which may affect long-term patency rates. Furthermore, these results are difficult to extrapolate to humans because endothelialization of vascular grafts occurs more readily in the canine and rabbit models used in the studies.⁹

Due to the numerous problems associated with the current techniques in tissue engineering of vascular structures there remains a need for improved tissue-engineering methods that would provide a reliable source of cells and long-term patency and avoid the invasive arterial or venous biopsies currently required for harvesting autologous cells.

### SUMMARY OF THE INVENTION

The present invention is directed to novel, less invasive and improved methods of tissue-engineering of constructs that require an endothelial surface such as blood vessels and heart valves. The constructs of the present invention possess long-term, patency. The method of the present invention is particularly suited for making small diameter vessels to replace clogged or damaged coronary blood vessels, for making trileaflet heart valve conduits to replace damaged or malformed pulmonic and aortic valves, and for other vascular structures that require an endothelial surface.

The method of the present invention is particularly suited for making tissue-engineered structures that require an endothelial surface, such as small diameter vessels, heart valves and components of heart valves such as leaflets or supports.

The method of the present invention includes the steps of isolating endothelial progenitor cells (EPC) and/or alpha-smooth muscle action (SMA) positive cells with a spindle morphology (referred to herein as "SMA⁺ spindle cells"), expanding the cells *in vitro*, seeding the cells on a suitable scaffold, and preconditioning the seeded scaffold under the conditions similar to those experienced *in vivo* to form a tissue-engineered construct such as a blood vessel, a heart valve, or a vascular graft. Importantly, the EPC grafts of the present invention exhibit contractile activity and nitric oxide-mediated vascular relaxation that is similar to native carotid arteries but which have not been reported previously for tissue-engineered small diameter grafts. These results indicate that EPCs can function similarly to arterial endothelial cells and thereby confer longer vascular graft survival.

Ideally, the cells used in the present invention are autologous and non-immunogenic. Most preferably, the cells are isolated non-invasively and from the patient's peripheral blood. However, other sources of cells such as bone marrow and umbilical cord blood are also acceptable for use in the present invention. The isolated cells are expanded *in vitro* and subsequently seeded onto a suitable scaffold such as a vascular graft. EPCs and SMA⁺ spindle cells may be seeded on the graft alone, or in combination. In the preferred embodiment, the seeded scaffold is preconditioned prior to implantation *in vivo* by being subjected to shear stress, e.g., in a bioreactor, so as to yield the maximum retention of EPCs on the scaffold. A suitable bioreactor would be capable of subjecting the seeded scaffold to conditions similar to those experienced in vivo, namely changes in flow and pressure. An intradevice fluid flow is preferably set to gradually increase from low pressure to high pressure over a period of several days. A pulsatile bioreactor is preferred (WO 00/07890).

Tissue-engineered structures of the present invention can be made of any suitable shape, including, but not limited to, solid porous scaffolds such as spheres, ellipsoids, two-dimensional structures such as disks, patches, sheets or films, as well as hollow porous substrates such as hollow spheres or ellipsoids, and open-ended tubes. In the preferred embodiments for tubular tissue-engineered constructs, the scaffolds comprise substantially tubular or cylindrical shapes, including tubular shapes with diameters which vary along the length of the scaffold. Thus, the scaffold of the invention can be, for example, a tubular scaffold of an appropriate diameter (preferably less than 5 mm in internal diameter), a non-cylindrical scaffold in the shape of a heart valve or a component of a heart valve such as a leaflet or a support.

The scaffold with necessary mechanical properties and bioabsorption profiles can be made from any biodegradable, biocompatible, synthetic polymeric materials (e.g., polyesters or polyanhydrides, optionally copolymerized with organic bases such as the basic amino acids), or proteinaceous polymers (e.g., collagen, elastin, fibronectin, laminin). The scaffolds for use in the present invention are synthetic or proteinaceous polymers having hydrophilic surfaces which promote cell-seeding. Such hydrophilic surfaces may be produced by hydrolyzing the surface of the substrate material to create free hydrophilic groups on the surface, or by otherwise modifying the surface with acylating, sulfonating, glycosylating, or other conjugating groups to increase hydrophilicity and/or provide better cell-adhesion characteristics. Various scaffold materials are disclosed in WO 99/01538, the disclosure of which is incorporated herein by reference. Additionally, the scaffolds of the present invention can be made of decellularized material from animal arterial vessels. The seeded scaffold may also be subjected to timed additions of growth factors and cytokines to stimulate specific steps in endothelial growth and differentiation.

The terms "construct", "seeded construct", "seeded scaffold", "graft" and "seeded graft" are used interchangeably herein and refer to the expanded EPCs being seeded on a suitable scaffold or graft.

The term "endothelial progenitor cells," EPCs, includes cells referred to as "circulating endothelial cells" (CEC) disclosed in U.S. Provisional Application 60/244,277, the disclosure of which is incorporated herein by reference.

It is to be understood that while the invention has been described in conjunction with the preferred specific embodiments thereof that the foregoing description as well as the examples that follow are intended to illustrate and not limit the scope of the invention. Other aspects, advantages and modifications within the scope of the invention will be apparent to those skilled in the art to which the invention pertains.

### BRIEF DESCRIPTION OF THE FIGURES

The accompanying drawings, which are incorporated in and constitute a part of this specification, illustrate embodiments of the invention and, together with the description, serve to explain the objects, advantages, and principles of the invention. In the drawings:

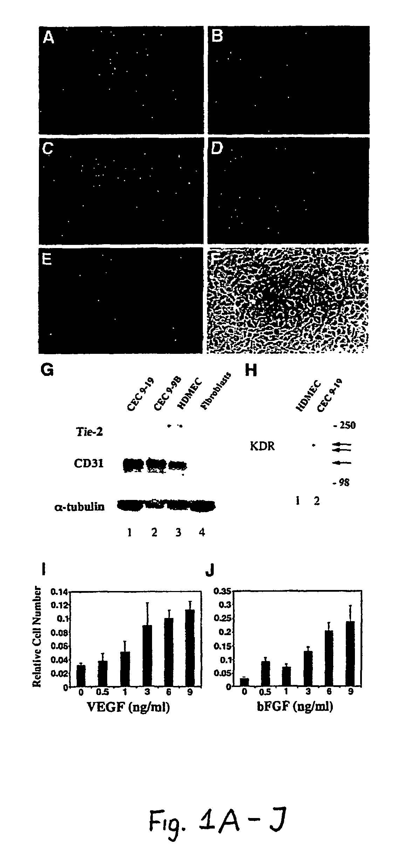

**Figures 1A-1H** illustrate morphologic and phenotypic properties of EPCs from peripheral blood EPCs exhibit typical cobblestone endothelial morphology (**Fig. 1A),** CD31 (**Fig. 1B),** von Willebrand factor **(Fig. 1C)** and incorporate aLDL (**Fig. 1D).** Unstimulated EPCs are negative for E-selectin expression (**Fig. 1E),** but LPS stimulated expression of E-selectin (**Fig. 1F). Figs. 1G** and **1H** show whole-cell extracts of two different EPC populations, EPC 9-19 and EPC 9-9B, HDMEC, and human fibroblasts immunoblotted with antibodies (shown on the left of each panel). Human fibroblast extracts serve as a negative control and HDMEC as the positive control, and α-tubulin served as a control for protein loading. The three arrows (**Fig. 1H)** indicate the fully glycosylated mature form of KDR (230 kDa), the partially glycosylated form of KDR (200 kDa), and unglycosylated form of KDR (150 kDa)⁴⁰. **Figs. 1I** and **1J** illustrate proliferation of EPCs in response to increasing concentrations of VEGF and BFGF, respectively after 72 hours of incubation with the growth factors.

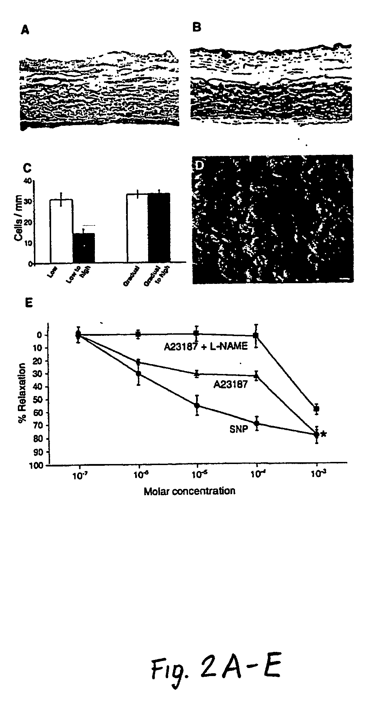

**Figures 2A-2E** illustrate results of pre-implantation studies with EPCs seeded on the decellularized porcine iliac matrix. **Fig. 2A** illustrates histology of the decellularized graft by hematoxylin and eosin stain (magnification 20X). Movat stain (**Fig. 2B)** showed the internal and external elastin layers (dark brown) (magnification 20X). **Fig. 2C** shows EPC density on the luminal surface of the decellularized grafts treated with low (1 dynes/cm²) or gradual (1 to 25 dynes/cm²) shear stress for 48-hours (open bars) and followed by 48 hour treatment with high (25 dynes/cm²) shear stress (black bars). **Fig. 2D** is a scanning electron microscopy of a EPC-seeded graft treated with gradual increases in shear stress for 48 hours and then an additional 48 hours of high shear stress showed a confluent layer of endothelial cells (magnification 880X, bar = 10 µ). **Fig. 2E** illustrates NO production by the EPC-seeded graft. In an organ chamber system, endothelial denuded guinea pig aortic ring was maximally contracted in the presence of U46619 and then relaxation was measured in the presence of increasing concentrations of calcium ionophore A23187 that was perfused directly into the EPC-seeded decellularized graft in the same organ chamber (▲). The relaxation effect mediated by A23187 was also measured in the presence of 10⁻³ M of L-NAME, an endothelial NO-synthase inhibitor (■). Relaxation was also recorded in the presence of increasing concentrations of SNP, an endothelial-independent NO donor (•). Data represent percent reduction in contraction and represent mean ± SEM derived from 4 grafts. *P<0.001 for dose response of A23187 vs. A23187 and L-NAME.

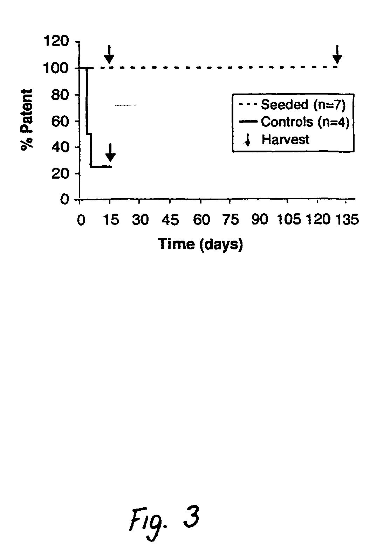

**Figure 3** illustrates the long term patency of vascular grafts seeded with EPCs (---) and control vascular grafts with no endothelial cells (-). Patency was defined by Doppler flow studies and confirmed by carotid duplex studies. The EPC-seeded grafts were harvested at 15 days (n=3) or at 130 days (n=4). The non-seeded grafts (controls) were harvested at 15 days (n=4).

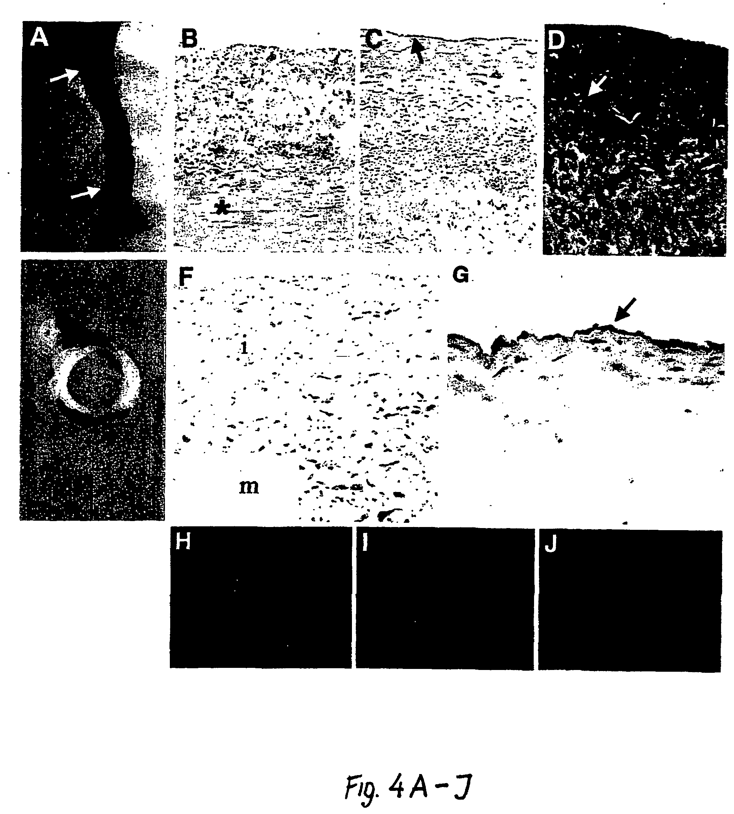

**Figures 4A-4I** depict post-implantation studies of the vascular grafts. **Fig. 4A** is an arteriogram of the EPC-seeded vascular graft 130 days after implantation. The location of the graft on the arteriogram was determined by staples at the sites of the anastomoses and is indicated by the two arrows. **Fig. 4B** depicts a seeded graft explanted at 15 days and shows a poorly organized thrombotic deposit overlying acellular graft media (asterisk). **Figs. 4C-F** depict a seeded graft explanted at 130 days. **Fig. 4C** shows a cellular intimal thickening as pannus from the adjacent arterial segments, infiltration of cells into the graft media, and confluent flattened cell monolayer lining the luminal surface of the graft (arrow). Fig. 4D shows a section stained for extracellular matrix demonstrates the preservation of the original anatomic features, especially elastin (black). The internal elastic membrane is denoted by a white arrow. Fig. 4E is a macroscopic view of the explanted 130-day EPC-seeded vessel, with 1mm scale. Fig. 4F depicts staining of the cells within the intimal layer (i and inset) for smooth muscle alpha-actin. The medial layer (**m**) is unstained for alpha-actin. **Fig. 4G** depicts staining of the cells overlying the intimal layer for vWF, denoted by arrow. Magnifications in **Figs. 4B, C** and **F** inset = 200X, in **Figs. 4D** and **4F** = 100X, and in **Fig. 4G** = 400X. **Fig. 4H** shows a pre-implant graft seeded with endothelial cells labeled with a fluorescent tracer. The 15 day (**Fig. 4I)** and 130 day **(Fig. 4J)** explanted grafts showed the presence of CM-DiI-labeled endothelial cells on the luminal surface of the graft. **Fig. 4G-I** are oriented with the lumen to the left.

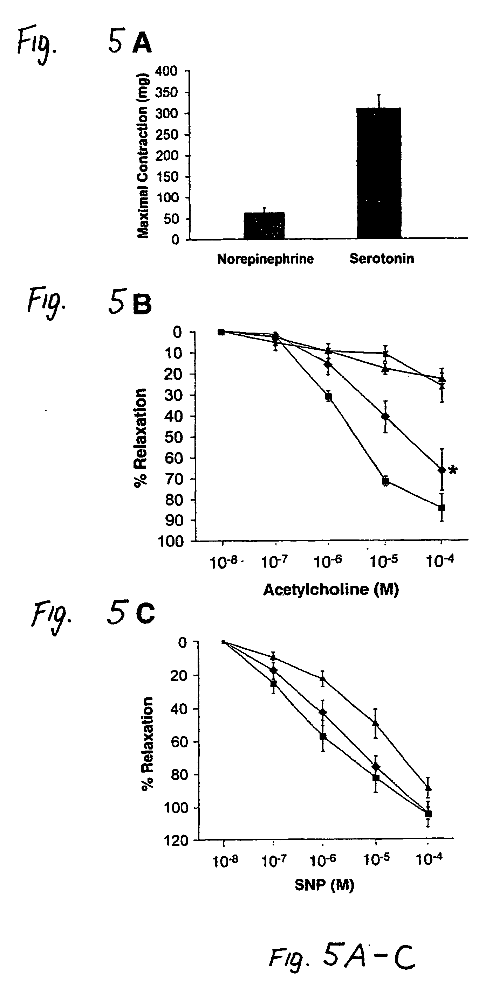

**Figures 5A-5C** illustrates a vasomotor responsiveness of explanted grafts at 130 days. **Fig. 5A** illustrates three mid-portion segments of each of the 130-day explant graft were tested for contractility activity in an organ chamber. Increasing doses of norepinephrine and serotonin, ranging from 10⁻⁹M to 10⁻⁴M, contracted the grafts and the maximal responses are shown at 10⁻⁴M concentration. **Fig. 5B** shows matched segments of EPC-seeded grafts (◆), carotid artery (■), and saphenous vein (▲) contracted with 10⁻⁴M of serotonin and relaxation was assessed in response to increasing concentrations of acetylcholine. The EPC-seeded graft was relaxed in the presence of acetylcholine and 10⁻³M of L-NAME (x). *P<0.00 1 for EPC-seeded graft vs the L-NAME attenuated response and saphenous vein and P=0.088 for EPC-seeded graft vs. carotid artery. **Fig. 5C** shows matched segments similar to **Fig. 5B** assessed for relaxation in the presence of SNP. Data represents percent reduction in contraction and mean ± SEM (n=12).

### DESCRIPTION OF THE INVENTION

We have surprisingly discovered that EPCs provide an ideal source of autologous endothelial cells for tissue-engineering of structures that require an endothelial surface because no sacrifice of a native vessel is necessary to obtain the cells. Furthermore, we found that the pre-conditioning of the EPC-seeded scaffold prior to its implantation *in vivo* creates a non-thrombogenic barrier which dramatically improves the patency rate of the resulting construct *in vivo*. Importantly, the EPC grafts of the present invention exhibit contractile activity and nitric oxide-mediated vascular relaxation that is similar to native carotid arteries but which have not been reported previously for tissue-engineered small diameter grafts. These results indicate that EPCs can function similarly to arterial endothelial cells and thereby confer longer vascular graft survival.

Endothelial cells are a crucial component of a normal vascular wall, providing an interface between the blood stream and surrounding tissue of the blood vessel wall. Endothelial cells also play an important role in physiological events including angiogenesis, inflammation, and prevention of thrombosis. In addition to the endothelial cells that comprise the vasculature, there is evidence for non-hematologic, endothelial progenitor cells (EPCs) circulating in the blood.¹⁰⁻¹⁴ EPCs migrate to regions of the circulation that have pathologic conditions affecting the injured endothelium, including traumatic and ischemic injury.¹⁵⁻²³ In normal adults, the concentration of EPCs in peripheral blood is 2-3 cells per milliliter, while levels are 3.5 fold higher in cord blood.^{14,23-24} Accordingly, cord blood is one preferred source of EPCs.

The preferred method of the present invention involves isolating EPCs from the patient, expanding them *in vitro*, seeding the cells onto an appropriate scaffold, and preconditioning the resulting construct under application of varying degrees of shear stress prior to implantation of the construct *in vivo*. Upon *implantation in vivo,* EPCs provide an autologous, non-thrombogenic luminal surface capable of maintaining patency for a substantial period of time, e.g., for at least 130 days. Moreover, the tissue-engineered constructs of the present invention develop into neovessels *in vivo* by 130 days and exhibit vasomotor activity to physiological stimuli.

In the preferred embodiment, EPCs are obtained non-invasively from relatively small amounts of the patient's peripheral blood, e.g., from about 15 cc to 450 cc of blood, particularly the leukocyte fraction of peripheral blood. However, other sources of EPCs including bone marrow and heterologous or autologous umbilical cord blood are also suitable for use in the present invention. The number of EPCs in peripheral blood can be increased by administering recruitment growth factors, e.g., GM-CSF and IL-3, to the patient. EPCs may also be isolated using antibodies that recognize EPC specific antigens such as AC133 and CD34 and receptors for vascular endothelial growth factor (VEGF) such as KDR/FIk-1 and VEGF R-2.

Preferably, EPCs are obtained by any blood-drawing procedure, such as venipuncture. Preferably, EPCs are partially purified using any known method, or, for example, a histopaque density gradient, and expanded. In the method of the present invention, EPC can be expanded *in vitro* by any of the methods known in the art. Suitable growth conditions and media for cells in culture are well known in the art. Cell culture media typically comprise essential nutrients, but also optionally include additional elements (e.g., growth factors, salts and minerals) which may be customized for the growth and differentiation of particular cell types. For example, "standard cell growth media" include Dulbecco's Modified Eagles Medium, low glucose (DMEM), with 110 mg/L pyruvate and glutamine, supplemented with 10-20% Fetal Bovine Serum (FBS) or 10-20% calf serum (CS) and 100U/ml penicillin. Other standard media include Basal Medium Eagle, Minimal Essential Media, McCoy's 5A Medium, and the like, preferably supplemented as above (commercially available from, e.g., JRH Biosciences, Lenexa, KS; GIBCO, BRL, Grand Island, NY; Sigma Chemical Co., St. Louis, MO). For example, to expand EPCs *in vitro*, a leukocyte fraction containing EPCs is plated onto fibronectin-coated plates as previously described¹². It is desirable to perform a serial transfer of the cell suspension to new fibronectin-coated plates to remove rapidly adherent hematopoietic cells. After approximately 21 to 28 days in culture, the number of outgrowth colonies typically ranges between 2 to 3 per 1x10⁷ total input cells. If allowed to continue growing, the outgrowth cells expands exponentially and reached 1.6x10⁹ cells by 3 weeks. Additionally, EPC progenitors can be mobilized *in vivo* by administration of recruitment growth factors, e.g., GM-CSF and IL-3, prior to removing the progenitor cells from the patient.

The outgrowth cells exhibit a cobblestone morphology that is characteristic of endothelial cells, as shown on Figure 1A. The endothelial phenotype can be confirmed by immunostaining with specific antibodies directed against endothelial markers. A representative EPC preparation uniformly expresses CD31 on the cell-cell membrane borders (Fig. 1B) and von Willebrand factor (vWF) in cytoplasmic granules similar to Weibel-Palade bodies (Fig. 1C). In addition, the EPCs incorporate acetylated low density lipoprotein (aLDL) (Fig.1D). However, E-selectin is minimally expressed in unstimulated cells (Fig. 1E), but upon stimulation with lipopolysaccharide (LPS), is significantly unregulated in the EPCs (Fig. 1F).

Another way to confirm the endothelial phenotype is to analyze whole cells extracts by immunoblot with specific antibodies to KDR/Flk-1, the vascular endothelial growth factor (VEGF) receptor-2 and Tie-2, the angiopoietin receptor (Fig. 1G and H). The EPCs tend to express equivalent amounts of Tie-2 and KDR when compared to human dermal microvascular endothelial cells (HDMEC). The response of EPCs to VEGF and basic fibroblast growth factor (bFGF) can be determined in cellular proliferation assays (Fig.1 I and J). Increasing VEGF and bFGF from 0 to 9 ng/ml results in approximately a 4-fold and 8-fold induction of cell number, respectively. The proliferative effect of VEGF and bFGF is relatively similar to that seen with HDMEC when using the same experimental assay²⁶. Consistent with the endothelial phenotype, the EPCs also form capillary tubes within 6-12 hours when plated on Matrigel. Important for tissue-engineering, the endothelial phenotype remains constant for at least 20 passages.

We have also identified a second cell type useful in making the tissue-engineered constructs of the present invention. When plated these cells exhibit a spindle-shaped morphology and express smooth muscle alpha actin (referred to "SMA spindle cells"). These cells can be obtained using the methodology set forth in Example 2. Immunofluorescent staining techniques can be used to characterize the cells.

Once EPCs are cultured from peripheral blood, they can be seeded onto a scaffold. Preferably, the scaffold comprises a biodegradable or bioerodable material, such as one which is slowly hydrolyzed under physiological conditions. Tissue-engineered structures of the present invention can be made of any suitable shape, including, but not limited to, solid porous scaffolds such as spheres, ellipsoids, two-dimensional structures such as disks, patches, sheets or films, as well as hollow porous substrates such as hollow spheres or ellipsoids, and open-ended tubes. In the preferred embodiments for tubular tissue-engineered constructs, the scaffolds comprise substantially tubular or cylindrical shapes, including tubular shapes with diameters which vary along the length of the scaffold. Thus, the scaffold of the invention can be, for example, a tubular scaffold of an appropriate diameter (preferably less than 5 mm in internal diameter), a decellularized vascular vessel, e.g., prepared from 4-mm diameter porcine iliac arteries of 4 to 5 cm in length can also be used.

Generally, any biocompatible, slowly hydrolyzable polymers may be employed. Preferred scaffold materials include polymeric materials such as polyesters, polyorthoesters, or polyanhydrides, including various polymers or copolymers of glycolic acid, lactic acid, or sebacic acid. More generally, preferred scaffold materials include polyesters of straight chain or branched, substituted or unsubstituted, saturated or unsaturated, linear or cross-linked, alkanyl, haloalkyl, thioalkyl, aminoalkyl, aryl, aralkyl, alkenyl, aralkenyl, heteroaryl, or alkoxy hydroxy acids (e.g., (COOH)(CH₂)ₙ(OH) or(COOH)(CRiRj)ₙ(OH), where n is an integer between about I and 20, and each Ri and Rj is independently selected from the group consisting of -H, -OH, -SH, -NH2, the halogens, the side chains of the naturally occurring amino acids, and any straight chain or branched, substituted or unsubstituted, saturated or unsaturated, low molecular weight (e.g., C₁-C₁₄) alkanyl, haloalkyl, thioalkyl, aminoalkyl, aryl, aralkyl, alkenyl, aralkenyl, heteroaryl, or alkoxy group, or a secondary or tertiary amine substituted with such groups) or polyanhydrides of straight chain or branched, substituted or unsubstituted, saturated or unsaturated, linear or cross-linked, alkanyl, haloalkyl, thioalkyl, aminoalkyl, aryl, aralkyl, alkenyl, aralkenyl, heteroaryl, or alkoxy dicarboxylic acids (e.g., (COOH)(CH₂)ₙ(COOH) or (COOH)(CRiRj)ₙ(COOH), where n is an integer between about I and 20, and each Ri and Rj is independently selected from the group consisting of -H, -OH, -SH,-NH₂, the halogens, the side chains of the naturally occurring amino acids, and any straight chain or branched, substituted or unsubstituted, saturated or unsaturated, low molecular weight (e.g., C₁-C₁₄) alkanyl, haloalkyl, thioalkyl, aminoalkyl, aryl, aralkyl, alkenyl, aralkenyl, heteroaryl, oralkoxy group, or a secondary or tertiary amine substituted with such groups). Polymers including mixtures of ester and anhydride bonds (e.g., copolymers of glycolic and sebacic acid) may also be employed. Thus, for example, preferred scaffold materials include polyglycolic acid polymers (PGA), polylactic acid polymers (PLA), polysebacic acid polymers (PSA), poly(lactic-co-glycolic)acid copolymers (PLGA), poly(lactic-co-sebacic) acid copolymers (PLSA), poly(glycolic-co-sebacic) acid copolymers (PGSA), etc.

Other biocompatible biodegradable polymers useful in the present invention include polymers or copolymers of caprolactones, carbonates, amides, amino acids, orthoesters, acetals, cyanoacrylates and degradable urethanes, as well as copolymers of these with straight chain or branched, substituted or unsubstituted, alkanyl, haloalkyl, thioalkyl, aminoalkyl, alkenyl, oraromatic hydroxy- or di-carboxylic acids. In addition, the biologically important amino acids with reactive side chain groups, such as lysine, arginine, aspartic acid, glutamic acid, serine, threonine, tyrosine and cysteine, or their enantiomers, may be included in copolymers with any of the aforementioned materials. The currently preferred biodegradable materials are PLA, PGA, and PLGA polymers. See, generally, U.S. Pat. Nos. 1,995,970; 2,703,361; 2,758,987; 2,951,828; 2,676,945; 2,683,136 and 3,531,561.

As an alternative to synthetic polymer scaffolds, porous scaffolds may be employed which comprise proteinaceous polymers. Such scaffolds are known in the art and have been used in the production of tissue-engineered constructs. For example, collagen gels have been used to produce vascular tissue constructs⁴³, and collagen sponges and meshes are now commercially available (e.g., from Ortec International, Inc., New York, New York). Such collagenous substrates, as well as similarly constructed substrates based on elastin, fibronectin, laminin, or other extracellular matrix or fibrillar proteins, may be employed in the methods and constructs of the present invention. Such proteinaceous polymer scaffolds may be in the form of fibrous meshes, as described above, or may be in the form of non-fibrous substrates such as sheets, patches, films, or sponges. In addition, these substrates may include proteinaceous polymers which have been modified by, for example, acylating, sulfonating, glycosylating, or otherwise conjugating reactive groups of the amino acid side chains with other moieties to increase hydrophilicity and/or provide better cell-adhesion characteristics. For example, the proteins may be acylated with dicarboxylic acid anhydrides to increase hydrophilicity, or may be conjugated to cell-adhesion peptides to increase the density or avidity of cell-seeding. Such proteinaceous polymers have the advantage that they are completely biological in nature and, therefore, will have reduced immunogenicity if syngeneic to the host.

In the preferred embodiment of the invention, after seeding a confluent monolayer of EPCs on the lumen of the scaffold, the seeded scaffold is preconditioned with variations in imposed shear stress to yield the maximal retention of EPCs prior to graft implantation *in vivo.* It has been previously shown that up to 80% of non-preconditioned endothelial cells on ePTFE grafts are dislodged after exposure to shear stress *in vitro* or after *vitro* or after implantation *in vivo*, and underscore the importance of pre-conditioning the endothelial cells on vascular grafts²⁷⁻²⁸.

Preferably, *in vivo* arterial conditions are simulated by subjecting the seeded construct to high shear stress (25 dynes/cm²). In the preferred embodiment, gradually increasing the shear stress from low (1 dynes/cm²) to high (25 dynes/cm²) over two days (Fig. 2C, open bar right) followed by two additional days of high shear stress results in the optimal cell density of the construct of about 32 cells/mm (Fig. 2C, black bar right). Scanning electron microscopy should reveal a confluent monolayer of endothelial cells (Fig. 2D). In contrast, pretreatment of the construct under conditions of low shear stress (1 dynes/cm²) for two days results in a cell density of 30 cells/mm (Fig. 2C, open bar left). And subsequently subjecting the construct to high shear stress (25 dynes/cm²) for another two days results in a cell density of 13 cells/mm, a reduction of nearly 50% (Fig. 2C, black bar left).

Once the pre-conditioning of the seeded construct is optimized, the function of the EPCs can be assessed by testing their ability to produce nitric oxide (NO) which plays a central role in normal vascular tissue homeostasis²⁹⁻³⁵. NO is not only a strong vasodilator but also an inhibitor of platelet aggregation, vascular smooth muscle proliferation, and leukocyte adhesion, all of which are strong anti-thrombotic and anti-atherogenic properties²⁹⁻³⁵. NO production by the EPC can be tested in a bio-assay using organ chamber methodology (Fig. 2E). Thus, endothelial-denuded segments of guinea pig aorta are contracted with U46619, a thromboxane analog, and then exposed to increasing concentrations of the calcium ionophore A23187 that is perfused through the EPC-seeded graft in the same organ chamber. Dose-dependent relaxation; of the aorta segments can be observed (Fig.2E, ▲). This dose-dependent relaxation can be significantly attenuated by adding 10⁻³ M of the endothelial NO-synthase inhibitor L-NAME (77.5% vs. 58.5%, P<.001, Fig.2E, **■**). When A23187 is perfused through the grafts that are not seeded with EPCs, the aortic segments would remain fully contracted. Exposing the aortic segments to sodium nitroprusside (SNP), an endothelial-independent NO donor, results in a dose-dependent relaxation, indicating normal smooth muscle function in the guinea pig aortic segments (Fig. 2E, •). These results confirm that after pre-conditioning, according to the method of the present invention, the EPCs on the graft produce NO.

The invention will be further characterized by the following examples which are intended to be exemplary of the invention:

### EXAMPLE 1

### Isolation of EPCs From Peripheral Blood.

EPCs were harvested from the internal jugular vein of 1 to 2 week old sheep¹. After the initial 5 cc of blood was discarded, 15 cc of blood was mixed with 100 units of heparin. The leukocyte fraction was obtained by centrifuging on a histopaque density gradient (Sigma, St. Louis, MO) for 30 minutes at 1000 x g using Accuspin tubes (Sigma). The cell pellet was resuspended in EBM-2 medium (Clonetics, San Diego, CA) with 20% fetal calf serum (Hyclone, Logan, UT) but without hydrocortisone and plated on fibronectin-coated plates. At 24-hour intervals for the next three days, the medium was transferred to new fibronectin-coated plates and thereafter the media was changed every 3 days. An aliquot of cells in the medium on day 3 were counted to determine input cells for final plating.

### Indirect Immunoflourescence

Indirect immunoflourescence was performed as previously described using antibodies to CD31 (Santa Cruz Biotechnology, Santa Cruz, CA), vWF (Dako, Carpenteria, CA), and E-selectin²⁶, and imaged for incorporation with fluorescent aLDL (Biomedical Technologies, Inc., Stoughton, MA). Cells were analyzed using a Zeiss Axiphot II fluorescence microscope and photographed using Ecktachrome p1600 color slide film.

### Western Blot Analysis

Cell lysates were prepared as described⁴¹ and separated by SDS-PAGE and transferred to Immobilon-P membrane. Anti-KDR and anti-Tie-2 antibodies (Santa Cruz Biotechnology, Santa Cruz, CA) were used to detect these endothelial specific proteins.

### Decellularized Graft Preparation

Freshly harvested porcine iliac blood vessels were placed in distilled water for one hour to remove blood elements. After harvesting, the iliac vessels are incubated with trypsin, and then processed by a 1% Triton X-100 (Sigma) and 0.1% ammonium hydroxide (Sigma) and shaken (120-RPM) at 4°C for 72 hours. The solution was changed every 24 hours. After cleaning, the vessels were lyophilized and cold gas sterilized. The resultant vessel graft should appear devoid of cellular content as seen by histological analysis (Fig. 2A), by the lack of RNA detected in a reverse transcription-polymerase chain reaction assay (data not shown), and by the lack of an immune response which can be determined by implanting 3mm² pieces of decellularized matrix into the peritoneal cavity of mice and observing the response.
Furthermore, Movat staining can be used to ascertain that the graft has a well-preserved extracellular matrix architecture, including internal and external elastic lamella (Fig. 2B).

### Isometric Studies

All isometric tension experiments were performed as previously described³⁷. Guinea pig aortic rings were suspended between 2 tungsten stirrups in an organ chamber bath of oxygenated 37°C Krebs solution. The vessels were increasingly stretched by 0.1 g increments to the optimal resting tension that produced a maximal response to 80 mmol/L KC1 and allowed to relax for one hour. EPC-seeded grafts were directly attached to the aortic ring. After U46619 contraction of the aortic ring, relaxation of the aortic ring was induced by stimulating NO production in the EPCs with increasing concentrations of Ca⁺² ionophore A23187. In addition, the graft response to A23187 and L-NAME at 10⁻³M was assessed and also in the presence of increasing concentrations of SNP. The EPC-seeded 130-day explant was also tested for vasomotor activity using the same experimental design by sectioning the explanted grafts into 5mm rings. Three rings were tested from each of the four 130-day explants. Response to increasing concentrations of norepinephrine and serotonin was evaluated in the grafts. After maximal contraction with 10^{-4M} of serotonin, relaxation responses to increasing concentrations of acetylcholine and SNP were determined in the grafts. Matched segments of ovine carotid artery and saphenous vein were used as the controls.

### EPC Labeling

EPCs were labeled with 1µg/ml of cell tracker CM-DiI (Molecular Probes, Eugene, OR), a fluorescent carbocyanine dye, for 20 min. at 37° and then washed three times with PBS. Subsequently, the labeled EPCs were seeded on the graft.

### Pre-conditioning of EPCs on the Graft

The grafts were seeded at the density of 1x10⁵ cells/cm² in a rotating dynamic seeder for 6 hours at 12 rpm. EPC-seeded acellular grafts were then placed in a laminar flow bioreactor and shear stress was calculated using Poiseuille's equation: τ=4 ηQ/ π r³, where τ= shear stress, η=fluid viscosity, Q=medium flow rate, and r=radius of the vessel. Shear stress was initiated either at 1 dynes/cm² for two days or gradually increased from 1 dynes/cm² to 25 dynes/cm² by increasing 3 dynes/cm² every 4 hrs over a 2 day period. Subsequently, all the grafts were subjected to 25 dynes/cm² for another two days. Three mid-portion segments of each graft were analyzed for confluency by examining 5µm histological sections and counting the number of nuclei per mm around the circumference at the luminal surface⁴².

### Statistical Analysis

All NO results are expressed as mean ± standard error of the mean (SEM). Statistical significance was evaluated using the unpaired Student's t-test for comparisons between two means.

### Surgical Implantation of the Grafts

Anesthetized sheep (30 ± 3 kg) underwent a common carotid artery exposure through a longitudinal incision in the mid neck. Heparin (100 IU/kg; Elkins-Sinn, Cherry Hill, NJ) was administered intravenously prior to arterial clamping, and the graft was placed as an end-to-end anastomosis to the common carotid artery using a 6.0 prolene suture (Ethicon, Somerville, NJ). Arterial flow was re-established and the closure was sutured by layers. Animal care and handling complied with the Guide for the Care and Use of Laboratory Animals published by National Institutes of Health.

### Explant Characterization

Tissue sections from the proximal and distal anastomoses and through the midportion of the graft were stained with hematoxylin and eosin. Smooth muscle cells and endothelial cells were identified by immunochemical analysis with antibodies to smooth muscle α-actin (Sigma) and vWF (Dako, Carpenteria, CA), respectively.

### Lone Term Patency of Vascular Grafts Seeded with EPCs and Control Vascular Grafts With No EPCs

To assess the efficacy of EPC-seeded grafts *in vivo,* implantation studies were performed in sheep. Autologous EPCs were isolated from one to two week old lambs, expanded in culture, and seeded onto a decellularized grafts. The grafts were then preconditioned over two days, gradually increasing shear stress from low to high. Seeded (n=7) and unseeded (n=4) grafts, 4 to 5 cm in length, were implanted to replace a segment of the carotid artery using two end-to-end anastomoses. The only anti-coagulant administered was daily aspirin (81 mg) for 7 days post-operatively. All grafts demonstrated unobstructed flow at the time of implantation. We performed ultrasound Doppler flow studies daily and carotid duplex studies two weeks postoperatively to assess patency, flow, and thrombus formation. Three out of four control, non-seeded grafts occluded from thrombus formation within 5 days of implantation (Fig. 3). One control graft was still patent when harvested at 15 days, but 50% of the lumen was obstructed by thrombus upon carotid duplex imaging and histologic analysis. All the grafts seeded with the EPCs were fully patent at the time of harvest, either 15 or 130 days after implantation (Fig. 3). A representative arteriogram of a 130-day EPC-seeded explant demonstrated a smooth luminal surface with no signs of thrombus, obstruction, or aneurysmal dilation (Fig. 4A). A macroscopic view of the 130 days EPC-seeded explant shown in Fig. 4E confirms the smooth surface of the vessel lumen.

### Post Implantation Studies of Vascular Grafts

Histological examination of the seeded grafts at 15 days revealed a confluent monolayer of endothelial cells (data not shown), however there were minimal areas of the lumen that had mild thrombus with cells concentrated at the thrombus-graft interface (Fig. 4B). The graft media was acellular. Histological examination of the entire length of the seeded grafts explanted at 130 days showed cellular intimal thickening as possible pannus from the adjacent arterial segments, infiltration of cells into the graft media, minimal adventitial inflammatory response, and a confluent flattened cell monolayer lining the lumenal surface of the graft (Fig. 4C). Staining with the Movat stain demonstrated preservation of the original anatomic features of extracellular matrix, especially elastin (Fig. 4D). The cells within the intimal layer stained positively for smooth muscle alpha-actin. a characteristic of smooth muscle cells (Fig. 4F), while those lining the luminal surface stained for vWF, a characteristic of endothelial cells (Fig 4G).

To determine if the seeded EPCs were still present on the graft at 130 days, the EPCs are labeled prior to seeding with a fluorescent lipophilic carbocyanine tracer that incorporates into cell membranes. Examination of graft tissue sections prior to implantation demonstrated a confluently labeled endothelial cell layer (Fig. 4H). Examination of the explanted grafts showed the presence of fluorescent EPC cells that covered approximately 80% and 10% of the lumen the graft, at 15 and 130 days respectively (Fig. 41 and 4J). Since the membrane-incorporated dye is diluted with every cell division. this result suggests a high proliferation rate of the EPCs on the graft. Alternatively, some of the seeded EPCs may have been detached from the graft *in vivo* or the tracer dye had diminished fluorescence at 130 days *in vivo.*

### Vasomotor Responsiveness of Explanted Grafts at 130 Days

Since smooth muscle cells were present in the grafts, the explanted graft was evaluated for contraction and relaxation reactivity (Fig. 5). The mid-segments of the 130 day explanted grafts were suspended in an organ chamber and contractility was measured in response to norepinephrine and serotonin. The 130 day explanted grafts contracted in a dose-dependent manner to norepinephrine and serotonin with a maximal response shown in Fig. 5A. In comparison to the explanted grafts, matched native carotid segments generated two-fold more force in response to serotonin and eight-fold more force contraction in response to norepinephrine. These results indicate that the vascular smooth muscle cells observed in the grafts are indeed functional and respond to two physiologically important vasoconstrictors.

Increased endothelial NO production is associated with higher patency rates of coronary artery bypass arterial conduits, for instance radial artery or internal mammary artery. In a previous set of experiments we demonstrated the capacity of the EPC-seeded graft to produce *NO in vitro* (Fig. 2E). To further assess the endothelial function of EPC-seeded grafts in an attempt to evaluate their long-term patency, the endothelial-dependent relaxation of explanted 130 day EPC-seeded grafts (◆) to that of native sheep carotid artery (■) and saphenous vein (▲) was compared. Segments of each vessel were pre-contracted with serotonin and exposed to increasing doses of acetylcholine inducing receptor-mediated NO production. Acetylcholine induced a dose-dependent relaxation in all three vessels (Fig. 5B). Maximal relaxation of the EPC-seeded graft to acetylcholine was significantly greater than saphenous vein segments (68% ± 10% vs 22 ±6%, P<.001),, but similar to carotid artery (68% ± 10% vs 81 ± 8%, P=.088) (Fig. 5B). The acetylcholine induced dose-dependent relaxation of the explanted grafts was attenuated in the presence of the endothelial NO-synthase inhibitor L-NAME (68% + 10% vs 22 ±6%, P<.001) (Fig. 5B, x). The three vessels responded equally to SNP (P=NS), an endothelial-independent NO donor, and achieved maximal relaxation, indicating normal vascular smooth muscle cell function of the explanted 130 day EPC-seeded grafts (Fig. 5C). Taken together, these results suggest that the endothelial-dependent and endothelial-independent vasomotor properties of the EPC-seeded grafts are very similar to that of native carotid arteries, and, therefore, potentially may have long-term patency rates.

### EXAMPLE 2

### Methods

### Experimental Design

Alpha-smooth muscle actin (SMA) positive cells with a spindle morphology were isolated from the peripheral blood of a newborn lamb. The cells were expaned on cell culture plates until a sufficient number was reached. The cells were then delivered in suspension to a biodegradable scaffold in the form of a similunar valve under rotating conditions. The cell-polymer scaffold was conditioned for two weeks in rotation prior to being transferred into a pulsatile flow simulator for an additional two weeks. Following the in vitro conditioning period, the tissue was harvested and analyzed for cellular and extracellular matrix protein content using histological staining, scanning electron microscopy and biochemical assays. Biochemical flexure testing was performed on one of the valves.

### Isolation Characterization and Expansion from Peripheral Blood

Fifteen milliliters of blood was drawn into a heparinized syringe from the jugular vein of a newborn sheep. The leukocyte fraction were isolated using a Ficoll density gradient centrifugation technique and plated on a fibronectin-coated a 60mm diameter polystyrene tissue culture plate (Falcon, Beckton-Dickinson, Franklin Lakes, NJ) in cell culture media (DMEM, Life Technologies, Grand Island, NY) supplemented with 10% fetal bovine serum (FBS) (Life Technologies, Grand Island, NY), 1% L-glutamine, penicillin, streptomycin (Life Technologies, Grand Island, NY), 2ng/ml basic fibroblastic growth factor (bFGF) Scios International, CA). The cell suspension from the original plate was serially transferred to a new fibronectin-coated plate every 24 hours for a total of three plates. Isolated colonies of cells with spindle morphology were seen on the first and second plates. Immunofluorescent staining using antibodies against smooth muscle cell proteins α-SMA (Sigma), desmin, calponin, myosin heavy chain, and endothelial cell surface receptor CD 31 was performed on the cell population. Cells were expanded in a humidified incubator at 5% CO₂ they reach 70% confluence in culture. At 70% confluence, the cells were trypsinized off of their original 60mm diameter plate using 0.25% Trypsin-EDTA (GibcoBRL, Life Technologies, Grand Island, NY) and passaged to a 150mm diameter plate. Thereafter, the cells were expanded in 150mm diameter plates and passaged at 70% confluence at a ratio of one dish to three. The media was replaced every 3 days or when the cells were passaged.

### Scaffold

A biodegradable scaffold was made from a composite of polyglycolic acid (PGA) (Albany International Research Co. Inc., Mansfield, MA) and poly-4-hydroxybuterate (P4HB) (Tepha Inc., Camhridge, MA). A 3cm in length by 19mm in diameter semilunar heart valve was fabricated around a sizing mould using a thermal processing technique (14).

### Cell Delivery onto and Conditioning of the Scaffold

Cells were isolated from the cell culture plates using 0.25% Trypsin-EDTA, and placed in a suspension of 100ml cell culture media (DMEM) supplemented with 10% fetal bovine serum, 1% L-glutamine, penicillin, streptomycin and 2ng/ml bFGF at a concentration of 2 million cells per 1 cm² scaffold. The cell-media-scaffold combination was placed in a standard closed hybridization flask and rotated in a hybridization oven at a rate of 4 rpms and a temperature of 37° C. Fresh cell culture media containing ascorbic acid 200µg/ml was replaced at a 24 hour interval for a period of two weeks. The cell-polymer construct was then placed in an open system pulsatile flow simulator for an additional two weeks. Ascorbic acid 200µg/ml was supplemented every two days into the flow simulator.

Immediately following the four week conditioning time period, the tissue-engineered valve was removed from the flow simulator and processed for analysis. The structural integrity of the valves was assessed histologically and by scanning electron microscopy (SEM). Biochemical assays were performed to quantify cellular and extracellular matrix (ECM) composition. Biomechanical flexure testing was performed on the leaflets.

### RESULTS

Cells stained positively for α-smooth muscle actin (SMA), calponin and desmin, but negatively for platelet endothelial cell adhesion molecule (PECAM-1). Histological staining and SEM confirmed cellular and ECM material throughout the full thickness of the construct (n=3). Biochemical analyses showed 14.74µg DNA/dry weight (SD± 3.66), 4.47µg collagen/dry weight (SD± 2.0) and 34.46 µg elastin/dry weight (SD± 3.94) of construct (n=3). Preliminary biomechanical flexure testing of the leaflets showed effective stiffness of the tissue comparable to normal valve tissue. CONCLUSION: Cells from peripheral blood can populate a valve scaffold and produce ECM proteins to form "tissue" which mechanically resembles a normal heart valve.

### REFERENCES

1. Heart and Stroke Facts: Statistical Supplement, American Heart Association. http://www.americanheart.org/statistics (2000).
2. J.V. Tu, *et al*., Use of cardiac procedures and outcomes in elderly patients with myocardial infarction in the United States and Canada. *N Engl J Med* **336,** 1500 (1997).
3. Weinberg, C.B. & Bell, E. A blood vessel model constructed from collagen and cultured vascular cells. *Science* **213**, 397 (1986).
4. L'Heureux, N., Paquet, S., Labbe, R., Germain, L. & Auger, F.A. A completely biological tissue-engineered human blood vessel. *FASEB* J. **12,** 47 (1998).
5. Niklason. L.E., *et al*. Functional arteries grown in vitro. *Science* 284, 489 (1999).
6. T. Huynh, *et al., Nature Biot.* Remodeling of an acellular collagen graft into a physiologically responsive neovessel. 17, 1083 (1999).
7. Schmidt, C.E. & Baier, J.E. Acellular vascular tissues: natural biomaterials for tissue repair and tissue engineering. *Biomaterials* 21, 2215 (2000).
8. Sullivan, S.J. & Brockbank, K.G.M. in *Principles of Tissue-Engineering*. 447-454, (ed. Langer, B. & Vacanti, J.) (Academic Press, New York; 2000).
9. Davids, L., Dower, T. & Zilla, P. in *Tissue Engineering of Prosthetic Vascular Grafts.* 3-45, (ed. Zilla, P. & Griesler, H.P.) (R.G. Landes Co, Austin, TX; 1999).
10. Asahara, T. *et al*. Isolation of Putative Progenitor Endothelial Cells for Angiogenesis. *Science* **275,** 964 (1997).
11. Yin. A. *et al*. AC133, a novel maker for human hematopoietic stem and progenitor cells. *Blood* **90**, 5002 (1997).
12. Shi, Q. *et al*. Evidence for circulating bone marrow-derived endothelial cells. *Blood* **92**, 362 (1998).
13. Gehling, U. *M. et al*. In vitro differentiation of endothelial cells from AC133 -positive progenitor cells. *Blood* **95**, 3106 (2000).
14. Lin, Y., Weisdorf, D.J., Solovey, A. & Hebbel, R.R. Origins of circulating endothelial cells and endothelial outgrowth from blood. J *Clin Invest* **105**, 71 (2000).
15. Takahashi, T. *et al*. Ischemia- and cytokine-induced mobilization, of bone marrow-derived endothelial progenitor cells for neovascularization. *Nat. Med*. **5,** 434 (1999).
16. Asahara, T. *et al*. Bone marrow origin of endothelial progenitor cells responsible for postnatal vasculogenesis in physiological and pathological neovascularization. *Circul. Res.* **85,** 221 (1999).
17. Asahara, T. *et al*. VEGF contributes to postnatal neovascularization by mobilizing bone marrow-derived endothelial progenitor cells. *EMBO J.* **18,** 3964 (1999).
18. Kalka, *C. et al*. Transplantation of *ex vivo* expanded endothelial progenitor cells for therapeutic neovascularization. PNAS **97,**3422 (2000).
19. Kalka, C. *et al*. Vascular endothelial growth factor r65 gene transfer augments circulating endothelial progenitor cells in human subjects. *Circul. Res.* **86,** 1198 (2000).
20. Kalka, C. *et al*. VEGF gene transfer mobilizes endothelial progenitor cells in patients with inoperable coronary disease. *Ann. Thorac. Surg.* 70, 829 (2000).
21. Bhattacharya, V. *et al*. Enhanced endothelialization and microvessel formation in polyester grafts seeded with CD34⁺ bone marrow cells, *Blood* 95, 581 (2000).
22. Crosby, J.R. *et al*. Endothelial cells of hematopoietic origin make a significant contribution to adult blood vessel formation. *Circul. Res.* **87,** 728 (2000).
23. Murohara, *et al*. Transplanted cord blood-derived endothelial precursor cells augment postnatal neovascularization. *J. Clin. Invest.* **105,** 1527 (2000).
24. Peichev, M. *et al*. Expression of VEGFR-2 and AC133 by circulating human CD34⁺ cells identifies a population of functional endothelial precursors. *Blood* **95,** 952 (2000).
25. Ortenwall, P., Bylock, A., Kjellstrom, B.T. & Risberg, B. Seeding of ePTFE carotid interposition grafts in sheep and dogs: Species-dependent results. *Surgery* **103,** 199 (1987).
26. Kraling, B.M. & Bischoff, J. A simplified method for growth of human microvascular endothelial cells results in decreased senescence and continued responsiveness to cytokines and growth factors. *In Vitro Cell. Dev. Biol.* **33,** 308 (1998).
27. Rosenman, J.E., Kempczinski, R.F., Pearce, W.H. & Silberstein, E.B. Kinetics of endothelial cell seeding. *J. Vasc. Surg.* **2,** 778 (1995).
28. Miyata, T. *et al*. Delayed exposure to pulsatile shear stress improves retention of human saphenous vein endothelial cells on seeded ePTFE grafts. *J Surg. Res.* **50,** 485 (1991).
29. Keaney Jr., J.F. & Vita, J.A. Atherosclerosis, oxidative stress, and antioxidant protection in endothelium-derived relaxing factor action. *Prog. Cardiovasc. Dis.* **38,** 129 (1995).
30. Quyyumi, A.A. *et al.* Nitric oxide activity in the human coronary circulation. Impact of risk factors for coronary atherosclerosis. *J. Clin. Invest.* **95,** 1747 (1995).
31 Azuma, H., Ishikawa, M. & Sekizaki, S. Endothelium-dependent inhibition of platelet aggregation. *Br. J. Pharmacol*. **88,** 411 (1986).
32. Radomski, M.W., Palmer, R.M. & Moncada, S. The role of nitric oxide and cGMP in platelet adhesion to vascular endothelium. *Biochem. Biophys. Res. Commun*. **148,** 1482 (1987).
33. Kubes, P., Kurose. 1. & Granger, D.N. NO donors prevent integrin-induced leukocyte adhesion but not P-selectin-dependent rolling in postischemic venules. *Am. J. Physiol*. **267,** H931 (1994).
34. Marks, D. S. *et al*. Inhibition of neointimal proliferation in rabbits after vascular injury by a single treatment with a protein adduct of nitric oxide. *J. Clin. Invest.* **96,** 2630 (1995).
35. Luscher, T.F. *et al*. Difference between endothelium-dependent relaxation in arterial and in venous coronary bypass grafts. *N. Eng. J. Med*. **319,** 462 (1988).
36. Pearson, P.J., Evora, P.R.B. & Schaff, H.V. Bioassay of EDRF from internal mammary arteries: Implications for early and late bypass graft patency. *J. Thorac. Surg.* **54,** 1078 (1992).
37. Shapira, O.M. *et al*. Enhanced nitric oxide-mediated vascular relaxation in radial artery compared with internal mammary artery or saphenous vein. *Circulation* **100,** 11-322 (1999).
38. Shimizu,K., *et al*. Host bone marrow cells are a source of donor intimal smooth muscle-like cells in the murine aortic transplant arteriopathy. *Circulation* **102,** 11-221 (2000).
39. M.Deutsch, J. Meinhart, P. Zilla, *Tissue Engineering of Prosthetic Vascular Grafts, P.* Zilla, H.P. Griesler, Eds. (R.G. Landes Co., Austin, TX, 1999), pp. 180-187.
40. B. Shen, *et al*. Homologous up-regulation of KDR/Flk-1 receptor expression by vascular endothelial growth factor *in vitro. J. Biol. Chem*. **273,** 29979 (1998).
41. Shen, B. Q. *et al*. Hepatocyte growth factor stimulates the differentiation of human tracheal epithelia in vitro. *Am. J. Phys.* **272,** L1115 (1997).
42. Dardik, A., Liu, A., Ballermann, B.J. Chronic in vivo shear stress stimulates endothelial cell retention on prosthetic vascular grafts and reduces subsequent in vivo neointimal thickness. *J. Vasc. Biol.* **29,** 157 (1999).
43. Weinberg and Bell, *Science* **231,** 397-400 (1986).

## Claims

1. A tissue-engineered vascular construct comprising a scaffold configured to form said construct and seeded with endothelial progenitor cells and/or SMA⁺ spindle cells capable of forming vascular tissue, said scaffold comprising a biodegradable material.

2. The tissue-engineered vascular construct of claim 1, wherein the scaffold is configured to form a tube.

3. The tissue-engineered vascular construct of claim 1, wherein the scaffold is configured to form a trileaflet heart valve.

4. A method for making a tissue-engineered vascular construct comprising the steps of:
(a) providing a substrate shaped to form the vascular construct, said substrate comprising a biodegradable material;
(b) contacting said substrate with endothelial progenitor cells and/or SMA⁺ spindle cells capable of adhering thereto and forming vascular tissue, thereby forming a primary cell-seeded construct;
(c) maintaining said primary cell-seeded construct for a first growth period in a fluid media suitable for growth of said cells and imparting stresses in the construct during said first growth phase to simulate the physiological conditions to be encountered by the construct once implanted to form the vascular construct.

5. The method of claim 4, wherein the step of imparting stresses comprises cyclical increases in pressure and flow within said construct

6. The method of claim 4, wherein step of imparting stresses comprises a gradual increase in shear stress.

7. The method of claim 4, wherein said biodegradable material is a polymer.

8. A tissue-engineered trileaflet heart valve produced by the method of claim 4, 5, or 6.

9. A tissue-engineered tubular construct produced by the method of claim 4, 5, or 6.

10. A tissue-engineered two-dimensional construct produced by the method of claim 4, 5, or 6.

## Patentansprüche

1. Aus Gewebe hergestelltes vaskuläres Konstrukt umfassend ein Gerüst, das zur Bildung dieses Konstrukts konfiguriert und mit zur Bildung von vaskulärem Gewebe befähigten Endothel Vorläuferzellen und/oder SMA⁺-Spindelzellen besiedelt ist, wobei das Gerüst ein biologisch abbaubares Material umfasst.

2. Aus Gewebe hergestelltes vaskuläres Konstrukt nach Anspruch 1, worin das Gerüst zur Bildung eines Rohres konfiguriert ist.

3. Aus Gewebe hergestelltes vaskuläres Konstrukt nach Anspruch 1, worin das Gerüst zur Bildung einer dreiblättrigen Herzklappe konfiguriert ist.

4. Verfahren zur Herstellung eines aus Gewebe hergestellten vaskulären Konstrukts umfassend die Schritte:
(a) Bereitstellung eines Substrats zur Bildung des vaskulären Konstrukts, wobei das Substrat ein biologisch abbaubares Material umfasst;
(b) in Kontakt bringen des Substrats mit zur Anhaftung daran und zur Bildung vaskulären Gewebes befähigten Endothel-Vorläuferzellen und/oder SMA⁺-Spindelzellen, wodurch ein mit primären Zellen besiedeltes Konstrukt entsteht;
(c) Aufrechterhalten dieses mit primären Zellen besiedelten Konstrukts während einer ersten Wachstumsphase in einem für das Wachstum dieser Zellen geeigneten flüssigen Kulturmedium und Ausüben von Belastungen innerhalb des Konstruktes während dieser ersten Wachstumsphase zur Simulation der physiologischen Bedingungen, denen das Konstrukt nach

## Revendications

1. Produit d'assemblage vasculaire obtenu à partir de tissu et comprenant un squelette configuré de manière à former le produit d'assemblage et ensemencé de cellules souches endothéliales et/ou de cellules fusiformes SMA⁺ aptes à former du tissu vasculaire, le squelette comprenant une substance biodégradable.

2. Produit d'assemblage vasculaire obtenu à partir de tissu, suivant la revendication 1, dans lequel le squelette est configuré pour former un tube.

3. Produit d'assemblage vasculaire obtenu à partir de tissus, suivant la revendication 1, dans lequel le squelette est configuré pour former une valvule cardiaque à trois valves.

4. Procédé de production d'un produit d'assemblage vasculaire obtenu à partir d'un tissu comprenant les stades dans lesquels :
a) on se procure un substrat conformé pour former le produit de construction vasculaire, ce substrat comprenant une substance biodégradable,
b) on met le substrat en contact avec des cellules souches endothéliales et/ou des cellules fusiformes SMA⁺ aptes à y adhérer et à former du tissu vasculaire, en formant ainsi un produit d'assemblage primaire ensemencé de cellules,
c) on maintient le produit d'assemblage primaire ensemencé de cellules pendant une première durée de croissance dans un milieu fluide approprié à la croissance des cellules et on applique des contraintes aux produits d'assemblage pendant la première phase de croissance pour simuler les états physiologiques auxquels le produit d'assemblage se trouvera confronté une fois implanté pour former le produit d'assemblage vasculaire.
